(19) Europäisches Patentamt / European Patent Office / Office européen des brevets

(11) **EP 4 281 756 B1**

(12) # FASCICULE DE BREVET EUROPEEN

(45) Date de publication et mention
de la délivrance du brevet:
**26.02.2025 Bulletin 2025/09**

(21) Numéro de dépôt: **22700973.5**

(22) Date de dépôt: **19.01.2022**

(51) Classification Internationale des Brevets (IPC):
**G01N 21/3504** *(2014.01)*   **G01J 3/427** *(2006.01)*

(52) Classification Coopérative des Brevets (CPC):
**G01N 21/274; G01J 3/28; G01J 3/427;
G01N 21/314; G01N 21/3504;** G01N 2021/3137

(86) Numéro de dépôt international:
**PCT/EP2022/051156**

(87) Numéro de publication internationale:
**WO 2022/157208 (28.07.2022 Gazette 2022/30)**

(54) **PROCEDE DE CALIBRATION D'UN CAPTEUR DE GAZ ET PROCEDE DE MESURE D'UN GAZ METTANT EN OEUVRE LA CALIBRATION**

VERFAHREN ZUR KALIBRIERUNG EINES GASSENSORS UND VERFAHREN ZUR MESSUNG EINES GASES MIT DER KALIBRIERUNG

METHOD FOR CALIBRATING A GAS SENSOR AND METHOD FOR MEASURING A GAS USING THE CALIBRATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **21.01.2021  FR 2100597**

(43) Date de publication de la demande:
**29.11.2023  Bulletin 2023/48**

(73) Titulaire: **Elichens
38040 Grenoble (FR)**

(72) Inventeur: **JALLON, Pierre
38040 GRENOBLE (FR)**

(74) Mandataire: **INNOV-GROUP
209 Avenue Berthelot
69007 Lyon (FR)**

(56) Documents cités:
EP-A1- 3 179 233       WO-A1-2018/229239
WO-A1-2019/145649     US-A1- 2013 301 052

EP 4 281 756 B1

**Description**

**DOMAINE TECHNIQUE**

Le domaine technique de l'invention est un procédé optique d'analyse d'un gaz, en mettant en oeuvre une source de lumière de type corps noir ou corps gris et en mesurant une absorption d'une onde lumineuse émise par la source de lumière.

**ART ANTERIEUR**

Le recours à des méthodes optiques pour l'analyse d'un gaz est fréquent. Des capteurs permettent de déterminer la composition d'un gaz en se basant sur le fait que les espèces composant le gaz présentent des propriétés spectrales d'absorption différentes les unes des autres. Ainsi, connaissant une bande spectrale d'absorption d'une espèce gazeuse, sa concentration peut être déterminée par une estimation de l'absorption de la lumière traversant le gaz, en utilisant la loi de Beer-Lambert. Ce principe permet une estimation de la concentration d'une espèce gazeuse présente dans le gaz.

Selon les procédés les plus courants, le gaz analysé s'étend entre une source de lumière et un photodétecteur, dit photodétecteur de mesure, ce dernier étant destiné à mesurer une onde lumineuse transmise par le gaz à analyser, l'onde lumineuse étant partiellement absorbée par ce dernier. La source de lumière est usuellement une source émettant dans l'infrarouge, la méthode utilisée étant usuellement désignée par le terme anglosaxon "NDIR détection", l'acronyme NDIR signifiant Non Dispersive Infra-Red. Un tel principe a été fréquemment mis en oeuvre, et est par exemple décrit dans les documents US5026992 ou WO2007064370.

Les procédés usuels comprennent généralement une mesure d'une onde lumineuse, dite onde lumineuse de référence, émise par la source, l'onde lumineuse de référence étant non absorbée, ou absorbée de façon négligeable, par le gaz analysé. La mesure de l'onde lumineuse de référence permet d'estimer l'intensité de l'onde lumineuse émise par la source, ou d'estimer l'onde lumineuse qui serait détectée par le photodétecteur de mesure en l'absence d'absorption par le gaz analysé. Cette technologie est désignée par le terme "double beam". La comparaison entre l'onde lumineuse en présence de gaz et l'onde lumineuse sans gaz permet de caractériser l'absorption du gaz. Il s'agit par exemple de déterminer une quantité d'une espèce gazeuse dans le gaz, selon la technologie désignée par le terme "NDIR par absorption".

L'onde lumineuse de référence est mesurée par un photodétecteur de référence. Il peut s'agir d'un photodétecteur de référence différent du photodétecteur de mesure, et agencé de façon à être disposé face à la source de lumière, le photodétecteur de référence étant associé à un filtre optique de référence. Le filtre optique de référence définit une bande spectrale de référence, dans laquelle le gaz à analyser ne présente pas d'absorption significative. Le document WO 2019/145649 A1 décrit un procédé de calibration d'un capteur de gaz utilisant une fonction de calibration obtenue à partir d'une pluralité de concentrations connues du gaz.

L'inventeur a constaté que l'utilisation d'une onde lumineuse de référence, pour estimer l'onde lumineuse qui serait détectée par le photodétecteur de mesure en l'absence d'absorption par le gaz analysé, peut être une source d'incertitude importante. Il propose un procédé permettant de surmonter cet inconvénient, de façon à améliorer la précision de la mesure.

**EXPOSE DE L'INVENTION**

Un premier objet de l'invention est un procédé de calibration d'un capteur de gaz, le capteur de gaz étant destiné à déterminer une concentration d'une espèce gazeuse dans un gaz, l'espèce gazeuse étant apte à absorber une lumière dans une bande spectrale d'absorption, le capteur de gaz comprenant :

- une enceinte, configurée pour contenir le gaz ;
- une source de lumière, un photodétecteur de mesure, et un photodétecteur de référence, la source de lumière étant configurée pour émettre une onde lumineuse incidente, l'onde lumineuse incidente se propageant à travers l'enceinte vers le photodétecteur de mesure et vers le photodétecteur de référence ;

le capteur de gaz étant tel que

- la source de lumière est configurée pour être parcourue par un courant électrique d'alimentation, de façon à porter la source de lumière à une valeur de température ;
- le photodétecteur de mesure est agencé pour mesurer une intensité, dite intensité de mesure, d'une onde lumineuse, émise par la source de lumière, transmise par le gaz contenu dans l'enceinte, dans une bande spectrale de mesure, comprenant la bande spectrale d'absorption ;
- le photodétecteur de référence est agencé pour mesurer une intensité de référence d'une l'onde lumineuse de référence émise par la source de lumière dans une bande spectrale de référence ;

le procédé comportant les étapes suivantes :

- (i) prise en compte d'une fonction de calibration, de préférence non linéaire, la fonction de calibration permettant d'estimer une intensité mesurée, dans la bande spectrale de mesure, par le photodétecteur de mesure, en l'absence d'espèce gazeuse, à partir d'une intensité de référence, mesurée dans la bande spectrale de référence, par le photodétecteur de référence, la fonction de calibration dépendant de paramètres;
- (ii) remplissage de l'enceinte par un gaz comportant une concentration nulle, ou considérée comme nulle, de l'espèce gazeuse ;
- (iii) mesure, par le photodétecteur de mesure, d'une intensité de mesure, dans la bande spectrale de mesure, et mesure, par le photodétecteur de référence, d'une intensité de référence, dans la bande spectrale de référence, l'étape (iii) étant répétée à différents instants de calibration, en modifiant, entre différents instants de calibration, le courant d'alimentation de la source ;
- (iv) à partir des mesures résultant de (iii), détermination des paramètres de la fonction de calibration.

L'étape (i) peut comporter une prise en compte d'un modèle paramétrique non linéaire de la fonction de calibration. Le modèle paramétrique dépend de paramètres qui sont déterminés lors de l'étape (iv).
L'étape (iv) peut comporter :

- iv-1) à partir de chaque intensité de référence mesurée respectivement à chaque instant de calibration, estimation d'une intensité de mesure résultant du photodétecteur de mesure en utilisant la fonction de calibration ;
- iv-2) comparaison pour chaque valeur du courant d'alimentation, de l'intensité de mesure estimée lors de iv-1) avec l'intensité de mesure mesurée par le photodétecteur de mesure ;
- iv-3) détermination des paramètres de la fonction de calibration minimisant les comparaisons résultant de iv-2).

Lors de l'étape (iii), le courant d'alimentation peut varier entre deux valeurs extrêmes correspondant respectivement à -15% et +15% d'un courant d'alimentation nominal.
Selon un mode de réalisation,

- l'étape (iii) est mise en oeuvre en disposant le capteur de gaz à différentes valeurs d'une température ambiante ;
- l'étape (iv) est mise en oeuvre en prenant en compte les mesures résultant de (iii) pour chaque valeur de température, de façon à obtenir, une fonction de calibration respectivement associée à chaque valeur de température ambiante.

Selon un mode de réalisation,

- l'étape (iii) est mise en oeuvre en disposant le capteur de gaz à différentes valeurs de température ambiante ;
- l'étape (iv) est mise en oeuvre en prenant en compte les mesures résultant de (iii) pour l'ensemble des valeurs de température, de façon à obtenir, une fonction de calibration commune à l'ensemble des valeurs de température ambiante.

Selon un mode de réalisation, un paramètre de la fonction de calibration forme un exposant appliqué à l'intensité de référence, le paramètre étant estimé lors de l'étape (iv).
Selon un mode de réalisation, un paramètre de la fonction de calibration est un paramètre de proportionnalité, définissant une proportionnalité entre l'intensité estimée, dans la bande spectrale de mesure, en l'absence d'espèce gazeuse, et l'intensité de référence à laquelle l'exposant est appliqué, le paramètre de proportionnalité étant estimé lors de l'étape (iv).
Selon un mode de réalisation, la fonction de calibration comporte un ratio d'un numérateur sur un dénominateur, de telle sorte que :

- le numérateur comporte un premier paramètre multiplicatif appliqué à l'intensité de référence ;
- le dénominateur comporte un deuxième paramètre multiplicatif appliqué à l'intensité de référence ;
- le premier paramètre multiplicatif et le deuxième paramètre multiplicatif sont estimés lors de l'étape (iv).

Un deuxième objet de l'invention est un procédé de mesure d'une quantité d'une espèce gazeuse présente dans un gaz, l'espèce gazeuse étant apte à absorber une lumière dans une bande spectrale d'absorption, le procédé comportant les étapes suivantes :

- a) disposition du gaz entre une source de lumière et un photodétecteur de mesure, la source de lumière étant configurée pour émettre une onde lumineuse incidente, l'onde lumineuse incidente se propageant à travers le gaz vers le photodétecteur de mesure, la source de lumière étant parcourue par un courant électrique d'alimentation, de façon à porter la source de lumière à une valeur de température ;
- b) illumination du gaz par la source de lumière ;
- c) mesure, par le photodétecteur de mesure, d'une intensité, dite intensité de mesure, d'une onde lumineuse transmise par le gaz, dans une bande spectrale de mesure, comprenant la bande spectrale d'absorption ;
- d) mesure, par un photodétecteur de référence d'une intensité, dite intensité de référence, d'une onde lumineuse de référence, l'onde lumineuse de référence étant émise par la source de lumière dans une bande spectrale de référence ;

les étapes b) à d) étant mises en oeuvre à un instant de mesure, le procédé comportant, à chaque instant de mesure :

- e) à partir de l'intensité de référence mesurée par le photodétecteur de référence, prise en compte d'une fonction de calibration, de façon à estimer une intensité d'une onde lumineuse qui serait détectée par le photodétecteur de mesure, dans la bande spectrale de mesure, en l'absence de l'espèce gazeuse ;
- f) estimation d'une quantité de l'espèce gazeuse, à partir de l'intensité de mesure mesurée lors de l'étape c), de l'intensité estimée lors de l'étape e) ;

le procédé étant caractérisé en ce que la fonction de calibration est établie au cours d'une phase de calibration, la phase de calibration étant effectuée en mettant en oeuvre les étapes (i) à (iv) d'un procédé selon le premier objet de l'invention.

La fonction de calibration peut être établie de telle sorte que :

- l'étape (iii) est mise en oeuvre en disposant le capteur de gaz à différentes valeurs de température ambiante ;
- l'étape (iv) est mise en oeuvre en prenant en compte les mesures résultant de (iii) pour chaque valeur de température, de façon à obtenir, une fonction de calibration respectivement associée à chaque valeur de température ambiante ;
- l'étape e) comporte :

  • une prise en compte d'une température ambiante, autour du capteur de gaz ;
  • une sélection de la fonction de calibration en fonction de la température ambiante.

Un troisième objet de l'invention est un capteur de gaz, destiné à déterminer une concentration d'une espèce gazeuse dans un gaz, l'espèce gazeuse étant apte à absorber une lumière dans une bande spectrale d'absorption, le capteur comprenant :

- une enceinte, configurée pour contenir le gaz ;
- une source de lumière, un photodétecteur de mesure, et un photodétecteur de référence, la source de lumière étant configurée pour émettre une onde lumineuse incidente, l'onde lumineuse incidente se propageant à travers l'enceinte vers le photodétecteur de mesure et vers le photodétecteur de référence ;

le capteur étant tel que :

- la source de lumière est parcourue par un courant électrique d'alimentation, de façon à porter la source de lumière à une valeur de température ;
- le photodétecteur de mesure est configuré pour mesurer une intensité, dite intensité de mesure, d'une onde lumineuse, émise par la source de lumière transmise par le gaz, contenu dans l'enceinte, dans une bande spectrale de mesure, comprenant la bande spectrale d'absorption ;
- le photodétecteur de référence est configuré pour mesurer une intensité de référence d'une l'onde lumineuse de référence émise par la source de lumière dans une bande spectrale de référence ;
- le capteur comporte une unité de traitement, programmée pour stocker une fonction de calibration, de préférence non linéaire, établie selon le premier objet de l'invention et pour appliquer la fonction de calibration à l'intensité de référence mesurée par le photodétecteur de mesure.

Selon un mode de réalisation, le capteur de gaz comporte un capteur de température, configuré pour mesurer une température ambiante à l'instant de mesure, l'unité de traitement étant programmée pour sélectionner la fonction de calibration en fonction de la température ambiante, parmi plusieurs fonction de calibration respectivement associées à différentes températures ambiantes.

L'invention sera mieux comprise à la lecture de l'exposé des exemples de réalisation présentés, dans la suite de la description, en lien avec les figures listées ci-dessous.

## FIGURES

La figure 1A représente un exemple de capteur de gaz permettant la mise en oeuvre de l'invention.

La figure 1B schématise un spectre d'émission d'une source de lumière de type corps noir.

Les figures 2A et 2B représentent respectivement une erreur de mesure de la concentration de $CO_2$ et de $CH_4$ en fonction d'une erreur quant à l'estimation de l'onde lumineuse atteignant le photodétecteur de mesure.

La figure 3 représente les principales étapes d'un procédé de calibration et de mise en oeuvre d'un capteur de gaz.

La figure 4A représente des mesures, effectuées au cours d'une calibration, d'une intensité d'une onde lumineuse atteignant le photodétecteur de mesure, dans la bande spectrale de mesure, en différents instants de calibration. La figure 4A montre également une estimation de l'intensité mesurée, dans la bande spectrale de mesure, à chaque instant de calibration, en fonction de l'intensité mesurée dans la bande spectrale de référence, par le photodétecteur de référence.

La figure 4B montre des erreurs quadratiques moyennes entre des estimations et des mesures de l'intensité de l'onde lumineuse atteignant le capteur de mesure, en prenant en compte différentes fonctions de calibration, pour différents capteurs de gaz.

La figure 5A représente des mesures, effectuées au cours d'une calibration, d'une intensité d'une onde lumineuse atteignant le capteur d'image ; dans la bande spectrale de mesure, en différents instants de calibration. La figure 5A montre également l'estimation de l'intensité mesurée, dans la bande spectrale de mesure, à chaque instant de calibration, en fonction de l'intensité mesurée dans la bande spectrale de référence.

La figure 5B montre des erreurs quadratiques moyennes entre des estimations et des mesures de l'intensité de l'onde lumineuse atteignant le capteur de mesure, en prenant en compte différentes fonctions de calibration, pour différents capteurs de gaz.

Les figures 6A et 6B montrent des exemples d'essais d'un capteur avec et sans mise en oeuvre de l'invention.

## EXPOSE DE MODES DE REALISATION PARTICULIERS

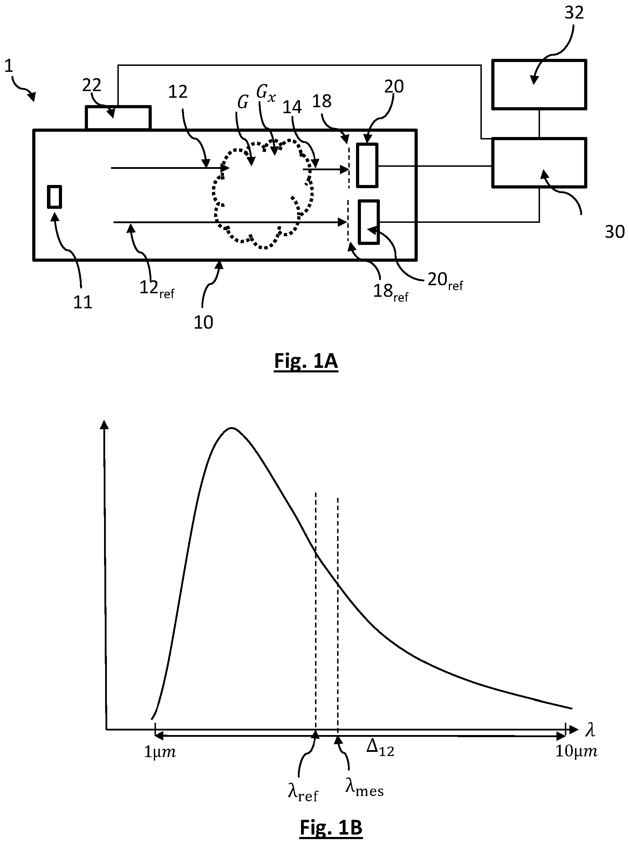

La figure 1A est un exemple d'un capteur 1 de gaz G. Le gaz G comporte une espèce gazeuse $G_x$ dont on cherche à déterminer une quantité $c_x(t)$, par exemple une concentration, à un instant de mesure $t$. Cette espèce gazeuse absorbe une part mesurable de la lumière dans une bande spectrale d'absorption $\Delta_x$.

Le capteur de gaz 1 comporte une enceinte 10 définissant un espace interne à l'intérieur duquel se trouvent :

- une source de lumière 11, apte à émettre une onde lumineuse 12, dite onde lumineuse incidente, de façon à illuminer un gaz G s'étendant dans l'espace interne. L'onde lumineuse incidente 12 s'étend selon une bande spectrale d'illumination $\Delta_{12}$.

- un photodétecteur 20, dit photodétecteur de mesure, configuré pour détecter une onde lumineuse 14 transmise par le gaz G, sous l'effet de l'illumination de ce dernier par l'onde lumineuse incidente 12. L'onde lumineuse 14 est désignée par le terme onde lumineuse de mesure. Elle est détectée, par le photodétecteur de mesure 20, dans une bande spectrale de mesure $\Delta_{mes}$ définie en fonction de la bande spectrale d'absorption $\Delta_x$ de l'espèce gazeuse $G_x$.

- un photodétecteur de référence $20_{ref}$, configuré pour détecter une onde lumineuse $12_{ref}$, dite de référence dans une bande spectrale de référence $\Delta_{ref}$. La bande spectrale de référence $\Delta_{ref}$ est une bande spectrale dans laquelle on considère que l'absorption de l'onde lumineuse 12 par le gaz G est négligeable.

La bande spectrale de référence $\Delta_{ref}$ est différente de la bande spectrale de mesure $\Delta_{mes}$.

La source de lumière 11 est configurée pour émettre l'onde lumineuse incidente 12, selon la bande spectrale d'illumination $\Delta_{12}$, cette dernière pouvant s'étendre entre le proche ultraviolet et l'infrarouge moyen, par exemple entre 200 nm et 10 $\mu$m, et le plus souvent entre 1 $\mu$m et 10 $\mu$m. La bande spectrale d'absorption $\Delta_x$ de l'espèce gazeuse $G_x$ analysée est comprise dans la bande spectrale d'illumination $\Delta_{12}$. La source de lumière 11 peut notamment être impulsionnelle, l'onde lumineuse incidente 12 étant une impulsion de durée généralement comprise entre 100 ms et 1 s. La source de lumière 11 peut notamment être une source de lumière de type filament suspendu et chauffé à une température comprise entre 400°C et 800°C. Son spectre d'émission, dans la bande spectrale d'illumination $\Delta_{12}$, correspond au spectre

d'émission d'un corps noir.

Le photodétecteur de mesure 20 est de préférence associé à un filtre optique 18, définissant la bande spectrale de mesure $\Delta_{mes}$ englobant tout ou partie de la bande spectrale d'absorption $\Delta_x$ de l'espèce gazeuse.

Dans l'exemple considéré, le photodétecteur de mesure 20 est une thermopile, apte à délivrer un signal dépendant de l'intensité de l'onde lumineuse détectée. De façon alternative, le photodétecteur de mesure peut être une photodiode ou un autre type de photodétecteur.

Le photodétecteur de référence $20_{ref}$ est disposé à côté du photodétecteur de mesure 20 et est de même type que ce dernier. Il est associé à un filtre optique, dit filtre optique de référence $18_{ref}$. Le filtre optique de référence $18_{ref}$ définit la bande spectrale de référence $\Delta_{ref}$ correspondant à une plage de longueurs d'onde non absorbées par l'espèce gazeuse considérée. La bande passante de référence $\Delta_{ref}$ est par exemple centrée autour de la longueur d'onde $\lambda_{ref}$ = 3.91 µm.

L'intensité $I_{mes}(t)$ de l'onde lumineuse 14 détectée par le photodétecteur de mesure 20, dite intensité de mesure, à un instant de mesure $t$, dépend de la concentration $c_x(t)$ à l'instant de mesure, selon la relation de Beer-Lambert :

$$abs(t) = 1 - \frac{I_{mes}(t)}{I_0(t)} = 1 - e^{-att_{mes}\, c_x(t)} \ (1)$$

où :

- $att_{mes}$ est une constante, qui correspond à l'atténuation de l'onde lumineuse dans la bande spectrale $\Delta_{mes}$ dans l'enceinte 10 pour une concentration unitaire de l'espèce gazeuse $G_x$. Cette constante peut être calculée sur la base d'un tracé de chemins optiques dans le capteur, entre la source de lumière et le photodétecteur de mesure et du coefficient d'absorptivité du gaz absorbant. Elle peut également être déterminée par étalonnage.
- $c_x(t)$ est la concentration de l'espèce gazeuse $G_x$ à l'instant $t$ ;
- $I_0(t)$ est l'intensité de l'onde lumineuse incidente, à l'instant $t$, qui correspond à l'intensité de l'onde lumineuse, dans la bande spectrale de mesure $\Delta_{mes}$, qui atteindrait le photodétecteur de mesure 20 en l'absence de gaz absorbant dans l'enceinte.

La comparaison entre $I_{mes}(t)$ et $I_0(t)$, prenant la forme d'un ratio $\dfrac{I_{mes}(t)}{I_0(t)}$, permet de définir une absorption $abs(t)$ générée par l'espèce gazeuse considérée à l'instant $t$. Connaissant $att_{mes}$, on peut déterminer $c_x(t)$.

L'expression (1) suppose une maîtrise de l'intensité $I_0(t)$ de l'onde lumineuse incidente 12 à l'instant de mesure $t$.

La figure 1B schématise un spectre d'émission d'une source de lumière 11 de type corps noir, obéissant à la loi de Planck :

$$L(\lambda, T) = \frac{2hc^2}{\lambda^5}\frac{1}{e^{\frac{hc}{\lambda K T}}-1} = \frac{2hc^2}{\lambda^5}\frac{1}{e^{\frac{\mu}{\lambda T}}-1} \ (2)$$

où

- $L(\lambda, T)$ est la luminance, dépendant de la longueur d'onde $\lambda$ et de la température de surface $T$ du corps noir ;
- h est la constante de Planck ;
- K est la constante de Boltzmann ;
- c est la vitesse de la lumière dans l'air ;
- $\mu = \dfrac{hc}{K}$ .
-

Le spectre d'émission S de la source de lumière 11 correspond à l'évolution de la luminance $L(\lambda, T)$ en fonction de $\lambda$, lorsque la source de lumière est portée à une température $T$. Généralement, la température $T$ est comprise entre 400°C et 800 °C.

On a représenté, sur la figure 1B, la bande spectrale d'illumination $\Delta_{12}$, de la source de lumière 11 s'étendant entre 1 µm et 10 µm. On a également représenté, par un trait en pointillés, la longueur d'onde de référence $\lambda_{ref}$ ainsi que la longueur d'onde de mesure $\lambda_{mes}$.

Ce type de source de lumière est particulièrement avantageux, car cela permet de moduler le spectre d'illumination S par une simple modulation de la température $T$ de la source. Ainsi, à chaque température $T$ est associé un spectre d'illumination S.

Il est connu que l'émissivité d'une source de lumière de type corps noir ou corps gris, peut varier avec le temps. La variation temporelle de l'émission de la source de lumière 11 est prise en compte par le photodétecteur de référence $20_{ref}$. Ce dernier est agencé pour détecter une onde lumineuse de référence $12_{ref}$, représentative de l'onde lumineuse incidente 12 émise par la source de lumière 11. L'onde lumineuse de référence $12_{ref}$ atteint le photodétecteur de référence $20_{ref}$ sans interagir avec le gaz G, ou sans interagir significativement avec ce dernier.

L'intensité de l'onde lumineuse de référence $12_{ref}$, détectée par le photodétecteur de référence $20_{ref}$, à l'instant de mesure $t$, est désignée par le terme intensité de référence $I_{ref}(t)$. A partir de $I_{ref}(t)$, connaissant le spectre d'émission de la source de lumière 11, on peut inférer l'intensité $\hat{I}_0(t)$ de l'onde lumineuse qui atteindrait le photodétecteur de mesure 20 en l'absence de gaz G. L'intensité de référence permet de corriger l'intensité de mesure $I_{mes}(t)$ pour prendre en compte les fluctuations temporelles rapides de puissance ainsi que le phénomène lent de vieillissement de la source de lumière 11.

Le dispositif comporte un microprocesseur 30, relié à une mémoire 32 comportant des instructions permettant la mise en oeuvre des étapes de procédé décrites ci-dessous. Le microprocesseur 30 est configuré pour recevoir un signal représentatif de l'intensité $I_{ref}(t)$ de l'onde lumineuse de référence $12_{ref}$, mesurée par le photodétecteur de référence $20_{ref}$ à chaque instant de mesure $t$. Le microprocesseur 30 estime l'intensité $\hat{I}_0(t)$ à partir de $I_{ref}(t)$.

A partir de $I_{mes}(t)$, on peut estimer l'absorption de l'onde lumineuse incidente selon l'expression :

$$abs(t) = 1 - \frac{I_{mes}(t)}{\hat{I}_0(t)} \quad (3).$$

. En utilisant l'expression (1), on obtient alors $c_x(t)$, de telle sorte que :

$$c_x(t) = -\frac{1}{att_{mes}} \ln\left(\frac{I_{mes}(t)}{\hat{I}_0(t)}\right) \quad (4)$$

Il est usuellement considéré que le ratio entre l'émissivité de la source de lumière 11, respectivement dans la bande spectrale de référence $\Delta_{ref}$ et dans la bande spectrale de mesure $\Delta_{mes}$ est constant. Selon une telle hypothèse, l'intensité $\hat{I}_0(t)$ est simplement estimée à partir de $I_{ref}(t)$ en supposant que le spectre théorique d'émission de la source de lumière est constant (i.e. la température $T$ est constante). Selon (2), on obtient $\hat{I}_0(t) \propto I_{ref}(t)$, où $\propto$ désigne l'opérateur « proportionnel à ».

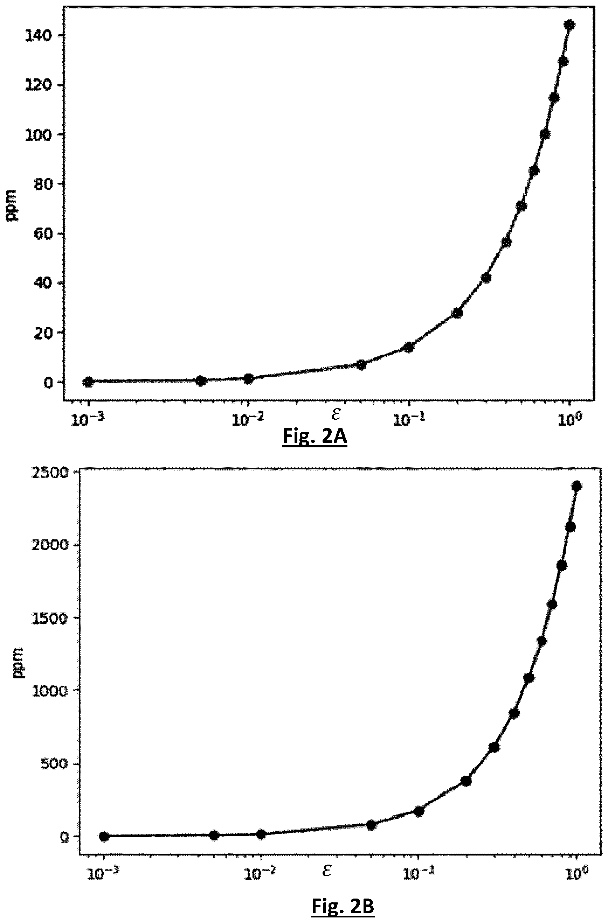

Cependant, l'inventeur a constaté qu'en utilisant l'expression (4), une faible erreur $\varepsilon$ dans l'estimation de $\hat{I}_0$ conduit, à une erreur élevée quant à l'estimation de $c_x$. Les figures 2A et 2B représentent l'incertitude associé à $c_x$ :

- lorsque $\lambda_{ref} = 3{,}91\ \mu m$ et lorsque $\lambda_{mes,x} = 4{,}26\ \mu m$, ce qui correspond à la longueur d'onde d'absorption de $CO_2$ : cf figure 2A ;
- lorsque $\lambda_{ref} = 3{,}91\ \mu m$ et lorsque $\lambda_{mes,x} = 3{,}25\ \mu m$, ce qui correspond à la longueur d'onde d'absorption de $CH_4$ : cf figure 2B.

Sur les figures 2A et 2B, l'axe des ordonnées représente une erreur associée à la concentration de $c_x$ (unité ppm) et l'axe des abscisses correspond à l'erreur $\varepsilon$. Pour $CO_2$ (figure 2A), des erreurs $\varepsilon$ de 0,1 et 0,2 entraînent respectivement une erreur de mesure de 14 ppm et 28 ppm. Pour $CH_4$ (figure 2B), des erreurs $\varepsilon$ de 0,1 et 0,2 entraînent respectivement une erreur de mesure de 100 ppm et 450 ppm. Les erreurs de mesure calculées pour $CO_2$ et $CH_4$, pour une même erreur d'estimation de $\hat{I}_0$, sont différentes car la pente de la courbe représentée sur la figure 1B est différente d'une part entre 3,91 $\mu m$ et 4,26 $\mu m$ et entre 3,91 $\mu m$ et 3,25 $\mu m$ d'autre part. De plus, les coefficients d'absorptivité des gaz $CO_2$ et $CH_4$ sont différents.

Compte tenu de (2), à chaque instant $t$, $I_{ref}(t)$ et $I_0(t)$ peuvent s'écrire :

$$I_{ref}(t) = \frac{a_{ref}I(t)}{e^{\left(\frac{\mu}{\lambda_{ref}T(t)}\right)}-1} \quad (6)$$

et

$$I_o(t) = \frac{aI(t)}{e^{\left(\frac{\mu}{\lambda_{mes,x}T(t)}\right)} - 1} \quad (7)$$

où :

- $I(t)$ est l'intensité lumineuse rayonnée par la source de lumière à l'instant $t$ ;
- $T(t)$ est la température de la source de lumière à l'instant $t$ ;
- $a_{ref}$ et $a$ dépendent des dispositions respectives de la source de lumière et des photodétecteurs de mesure et de référence ;
- $\mu$ est une constante définie en lien avec l'expression (2).

La température de la source T est une grandeur importante, car selon (2), elle conditionne le spectre d'émission de la source de lumière, tel que représenté sur la figure 1B. L'estimation de $\hat{I}_0$ à partir de $I_{ref}$ dépend du spectre d'émission de la source de lumière 11, donc de la température T.

Un aspect important de l'invention est de définir une fonction de calibration f permettant, à partir d'une mesure de $I_{ref}$, dans la bande spectrale de référence $\Delta_{ref}$, une estimation $\hat{I}_0$ précise de $I_0$, dans la bande spectrale de mesure $\Delta_{mes}$. Il est important que l'estimation $\hat{I}_0$ puisse être robuste à l'égard de variations de température T de la source de lumière.

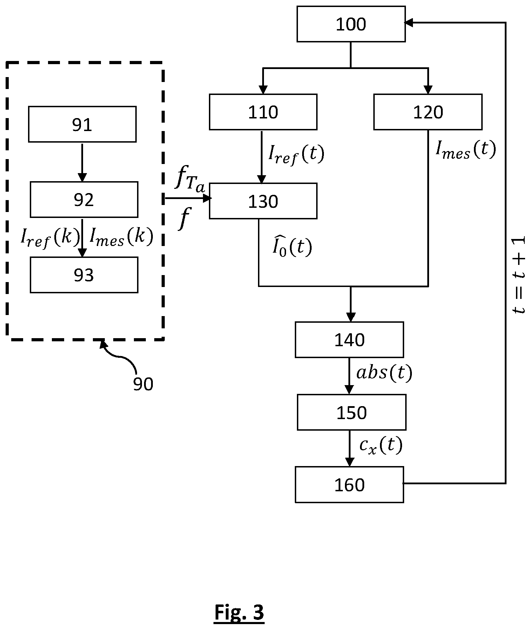

La figure 3 schématise les principales étapes de mise en oeuvre de l'invention. Au cours d'une phase de calibration 90, la fonction de calibration est définie. La phase de calibration comporte les étapes 91 à 93. La fonction de calibration résultant de la phase de calibration est ensuite utilisée lors d'une phase d'utilisation du capteur de gaz : étapes 100 à 160.

On va tout d'abord décrire la phase de calibration 90. La phase de calibration est effectuée durant plusieurs instants de calibration k.

Etape 91 : Prise en compte d'un modèle analytique paramétrique de la fonction de calibration $f$.

Au cours de cette étape, on définit un modèle analytique de la fonction de calibration. Le modèle analytique est défini par un ou plusieurs paramètres $\theta$. La grandeur $\theta$ correspond au paramètre du modèle ou à l'ensemble des paramètres du modèle.

Un premier modèle analytique, simple, est un modèle proportionnel. La fonction de calibration f est telle que :

$$\hat{I}_0 = f\left(I_{ref}\right) = Z I_{ref} \ (8).$$

Z est un coefficient réel positif. Selon ce modèle, $\theta = Z$

Un deuxième modèle analytique, plus raffiné, est obtenu est supposant que :

- la puissance de la source $I(k)$ est constante mais la température $T(k)$ varie, ce qui entraîne une variation de $I_{ref}(k)$ ;
- le terme $e^{\left(\frac{\mu}{\lambda_{ref}T(k)}\right)}$ est grand devant 1.

Ce modèle est :

$$\hat{I}_0 = f\left(I_{ref}\right) = Z\left(I_{ref}\right)^{\alpha} \ (9),$$

où Z et $\alpha$ sont des réels positifs.

Ce modèle résulte de simplifications des équations (6) et (7) selon lesquelles :

$$I_{ref}(k) = \frac{a_{ref}I(k)}{e^{\left(\frac{\mu}{\lambda_{ref}T(k)}\right)} - 1} \simeq \frac{a_{ref}I(k)}{e^{\left(\frac{\mu}{\lambda_{ref}T(k)}\right)}},$$

ce qui donne :

$$\frac{1}{T(k)} = \frac{\lambda_{ref}}{\mu}\ln\left(\frac{a_{ref}I(k)}{I_{ref}(k)}\right) \ (10)$$

Et de même :

$$I_o(k) = \frac{aI(k)}{e^{\left(\frac{\mu}{\lambda_{mes}T(k)}\right)} - 1} \simeq \frac{aI(k)}{e^{\left(\frac{\mu}{\lambda_{mes}T(k)}\right)}} \ (11)$$

La combinaison de (10) et (11) donne :

$$I_o(k) = \frac{aI(k)}{\exp\left(\frac{\mu}{\lambda_{mes}}\frac{\lambda_{ref}}{\mu}\ln\left(\frac{a_{ref}I(k)}{I_{ref}(k)}\right)\right)} = Z\left(I_{ref}(k)\right)^{\frac{\lambda_{ref}}{\lambda_{mes}}} = Z\left(I_{ref}(k)\right)^{\alpha} \quad (12)$$

Selon le modèle de l'expression (9), $\theta = (Z, \alpha)$
$\alpha$ peut être prédéterminé ou déterminé au cours de la calibration.

Un troisième modèle analytique peut être :

$$\hat{I}_0 = f(I_{ref}) = \frac{Z\left(1 + \xi(I_{ref}(k) - I_{ref}(k=0))\right)}{\left(1 + \sigma\frac{\left(1 + \xi\left(I_{ref}(k) - I_{ref}(k=0)\right)\right)}{I_{ref}(k)}\right)^{\gamma} - 1} \quad (13)$$

Le troisième modèle analytique est obtenu en considérant les grandeurs :

$$\tilde{I}_0(k) = \frac{I_0(k)}{I_0(k=0)} \quad (14) \;;$$

$$\tilde{I}_{ref}(k) = \frac{I_{ref}(k)}{I_{ref}(k=0)} \quad (15) \;;$$

$$\text{et } \tilde{I}(k) = \frac{I(k)}{I(k=0)} \quad (16)$$

k=0 désigne un instant de référence choisi de manière arbitraire. En général on choisira un instant où la source est alimenté à sa tension nominale.

Compte tenu de (10), (15) et (16),

$$\tilde{I}_{ref}(k) = \tilde{I}(k)\frac{e^{\left(\frac{\mu}{\lambda_{ref}T(k=0)}\right)} - 1}{e^{\left(\frac{\mu}{\lambda_{ref}T(k)}\right)} - 1} \quad (17)$$

A partir de (17), on peut écrire :

$$T(k) = \frac{\mu}{\lambda_{ref}} \cdot \frac{1}{\ln\left(1 + \sigma\frac{\tilde{I}(k)}{I_{ref}(k)}\right)} \quad (18)$$

avec

$$\sigma = I_{ref}(k=0)\left(e^{\left(\frac{\mu}{\lambda_{ref}T(k=0)}\right)} - 1\right) \quad (19)$$

Compte tenu de (11), (14) et (16)

$$\tilde{I}_0(\mathrm{k}) = \tilde{I}(\mathrm{k}) \frac{e^{\left(\frac{\mu}{\lambda_{mes}T(k=0)}\right)} - 1}{e^{\left(\frac{\mu}{\lambda_{mes}T(k)}\right)} - 1} \quad (20)$$

En utilisant (18), on obtient :

$$\tilde{I}_0(\mathrm{k}) = \frac{Z\tilde{I}(\mathrm{k})}{\left(1 + \sigma \frac{\tilde{I}(\mathrm{k})}{I_{ref}(\mathrm{k})}\right)^{\frac{\lambda_{ref}}{\lambda_{mes}}} - 1} \quad (21)$$

En considérant que l'intensité lumineuse émise par la source de lumière varie peu, on peut linéariser le terme $\tilde{I}(\mathrm{k})$ par l'expression :

$$\tilde{I}(\mathrm{k}) = 1 + \xi\left(I_{ref}(k) - I_{ref}(k = 0)\right) \quad (22)$$

La combinaison de (21) et (22) donne :

$$\hat{I}_0(\mathrm{k}) = \frac{Z\left(1 + \xi\left(I_{ref}(k) - I_{ref}(k = 0)\right)\right)}{\left(1 + \sigma \frac{1 + \xi\left(I_{ref}(k) - I_{ref}(k = 0)\right)}{I_{ref}(\mathrm{k})}\right)^{\frac{\lambda_{ref}}{\lambda_{mes}}} - 1} \quad (23)$$

Afin de prendre en compte la largeur des bandes spectrales s'étendant respectivement autour des longueurs d'onde $\lambda_{ref}$ et $\lambda_{mes}$, l'expression (23) peut être généralisée à :

$$\hat{I}_0(\mathrm{k}) = \frac{Z\left(1 + \xi\left(I_{ref}(k) - I_{ref}(k = 0)\right)\right)}{\left(1 + \sigma \frac{1 + \xi\left(I_{ref}(k) - I_{ref}(k = 0)\right)}{I_{ref}(\mathrm{k})}\right)^{\gamma} - 1} \quad (24)$$

Selon le modèle des expressions (13) ou (24), $\theta = (Z, \gamma, \varsigma, \sigma)$ ou $\theta = (Z, \varsigma, \sigma)$ lorsqu'on considère le modèle résultant de l'expression (23). Z, y, ç, $\sigma$ sont des réels positifs.

Un quatrième modèle est tel que :

$$\hat{I}_0(\mathrm{k}) = f(I_{ref}(k)) = \frac{ZI_{ref}(k)}{1 + \beta\left(I_{ref}(k) - I_{ref}(k = 0)\right)} \quad (25)$$

Selon ce modèle $\theta = (Z, \beta)$

<u>Etape 91</u> mesures de calibration.

Au cours de cette étape, le capteur de gaz est exposé à un gaz ne comportant pas l'espèce gazeuse $G_x$, ou selon une quantité négligeable. Ainsi, lors de chaque mesure, $I_{mes}(k) = I_0(k)$. Des mesures de $I_{ref}(k)$ et de $I_{mes}(k)$ sont effectuées à différents instants de calibration $k$.

Entre différents instants de calibration, le courant d'alimentation de la source de lumière 11 est modifié. La tension aux bornes de la source de lumière 11 est ainsi modifiée. Cela permet de porter la source de lumière à différents niveaux de température. Pour une même valeur de courant, on effectue différentes mesures de $I_{mes}(k)$ et $I_{ref}(k)$.

Généralement, la source de lumière est associée à une valeur de courant nominale, qu'il s'agisse d'une intensité ou d'une tension. De préférence, durant la calibration, le courant d'alimentation varie entre deux valeurs extrêmes, respectivement comprises entre -15% et +15% de la valeur nominale.

<u>Etape 92</u> : estimation des paramètres $\theta$

A partir des mesures de calibration $I_{mes}(k)$ et $I_{ref}(k)$, les paramètres du modèle paramétrique sont estimés en minimisant une fonction de coût, représentant un écart entre :

- chaque intensité mesurée $I_{mes}(k)$, qui est égale à $I_0(k)$) ;
- chaque estimation de l'intensité $\hat{I}_0(k)$ à partir de $I_{ref}(k)$ à partir de la fonction de calibration $f$ : $\hat{I}_0(k) = f(I_{ref}(k))$.

Ainsi,

$$\hat{\theta} = \underset{k}{\arg\min} \sum_k \left[ I_{mes}(k) - \hat{I}_0(k) \right]^2 \quad (30)$$

$\hat{\theta}$ est l'estimation des paramètres $\theta$.

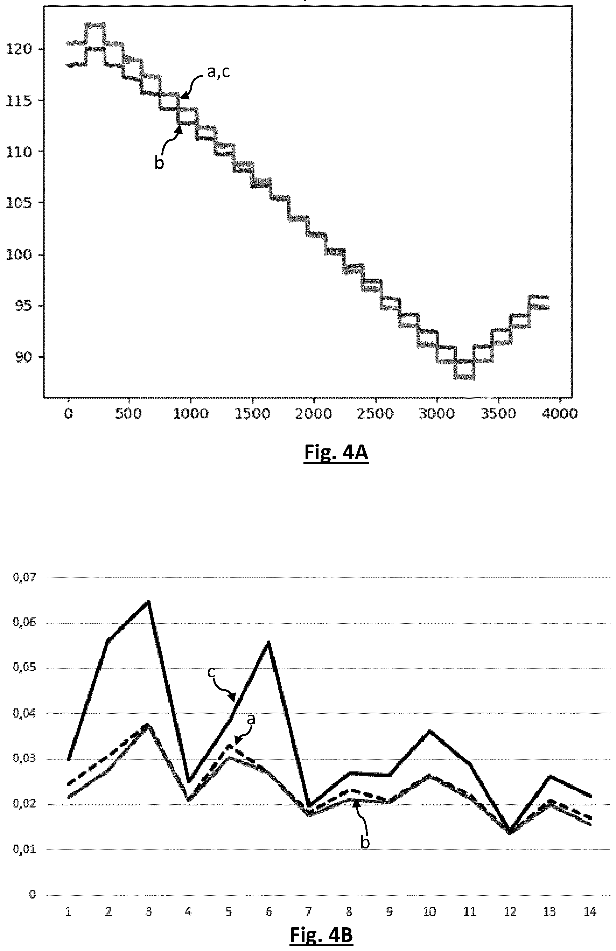

La figure 4A représente un résultat de mesures de calibration effectuées sur un capteur, en prenant en compte vingt et une différentes valeurs de courant d'alimentation de la source de lumière. Les valeurs du courant d'alimentation ont été variées entre deux valeurs extrémales, correspondant respectivement à -15% et +15% de la valeur de courant nominale de la source de lumière. Le capteur était tel que décrit dans EP3593119. On a effectué 4000 mesures à raison d'une mesure par seconde, l'espèce gazeuse mesurée étant $CO_2$. A chaque instant de calibration $k$, on a mesuré $I_{mes}(k)$ et $I_{ref}(k)$. A partir de l'ensemble des valeurs mesurées, on a estimé les paramètres $\theta$ d'une fonction de calibration en considérant les modèles paramétriques respectivement explicités dans les expressions (8), (9), (23), et (25). La figure 4A montre, en fonction du temps (axe des abscisses - unité seconde).

- la mesure de $I_{mes}(k)$, sachant que $I_{mes}(k) = I_0(k)$ : courbe a ;
- une estimation de $\hat{I}_0(k) = f(I_{ref}(k))$, selon l'expression (8) : courbe b ;
- une estimation de $\hat{I}_0(k) = f(I_{ref}(k))$, selon l'expression (25) : courbe c ;

Sur la figure 4A, l'axe des abscisses correspond aux instants de calibration k. unité : secondes. L'axe des ordonnées correspond à $I_0(k)$ ou $\hat{I}_0(k)$ - unité mV.

Une calibration telle que décrite en lien avec la figure 4A a été effectuée sur 14 capteurs différents. La figure 4B représente, pour chaque capteur (axe des abscisses), une erreur quadratique moyenne (MSE Mean Squared Error : axe des ordonnées), la MSE étant obtenue selon l'expression :

$$MSE = \frac{1}{N_k} \sum_k \left| I_{mes}(k) - \hat{I}_0(k) \right|^2 (31)$$

Sur la figure 4B, les courbes a, b, correspondent respectivement aux modèles paramétriques établis respectivement selon les expressions (9) et (23). La courbe c) correspond à un modèle linéaire (équation (8)), préétabli, avec Z = 1,11977. Il apparaît que les modèles a) et b) semblent particulièrement pertinents.

Une nouvelle calibration a été effectuée en réduisant la variation du courant d'alimentation des sources de lumière. Les valeurs du courant d'alimentation ont été variées entre deux valeurs extrémales, correspondant respectivement à 5% et +5% de la valeur de courant nominale de la source de lumière. Dans cet intervalle, on a pris en compte 7 différentes valeurs de courant d'alimentation de la source de lumière.

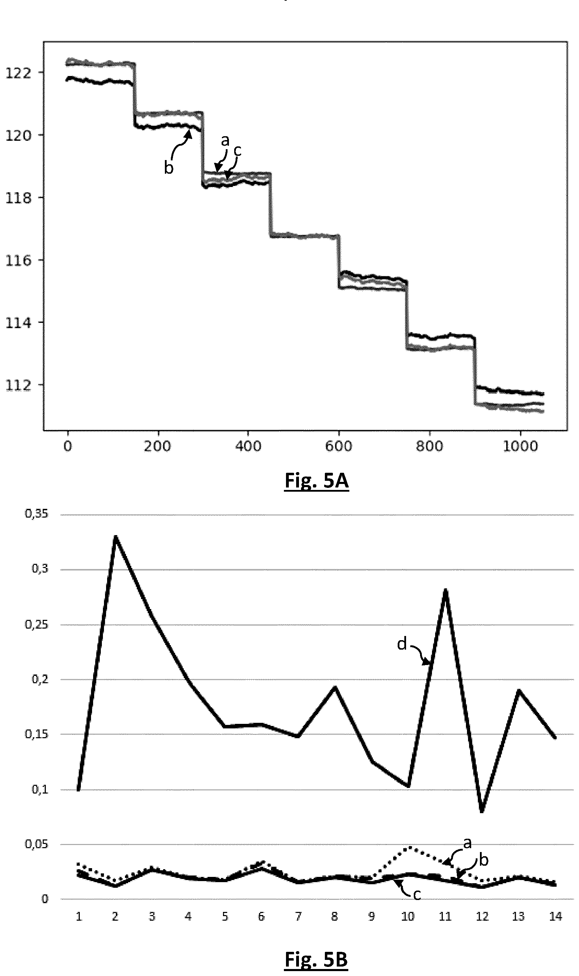

On a effectué 1050 mesures à raison d'une mesure par seconde. A chaque instant de calibration $k$, on a mesuré $I_{mes}(k)$ et $I_{ref}(k)$. A partir de l'ensemble des valeurs mesurées, on a estimé les paramètres $\theta$ d'une fonction de calibration en considérant les modèles paramétriques respectivement explicités dans les expressions (8), (9), (23), et (25). La figure 5A montre, en fonction du temps (axe des abscisses - unité seconde).

- la mesure de $I_{mes}(k)$, sachant que $I_{mes}(k) = I_0(k)$ : courbe a ;
- une estimation de $\hat{I}_0(k) = f(I_{ref}(k))$, selon l'expression (8) : courbe b ;
- une estimation de $\hat{I}_0(k) = f(I_{ref}(k))$, selon l'expression (25) : courbe c ;

Sur la figure 5A, l'axe des abscisses correspond aux instants de calibration k. unité : secondes. L'axe des ordonnées correspond à $I_0(k)$ ou $\hat{I}_0(k)$ - unité mV.

Une calibration telle que décrite en lien avec la figure 5A a été effectuée sur 14 capteurs différents. La figure 5B représente, sur pour chaque capteur (axe des abscisses), une erreur quadratique moyenne (MSE Mean Squared Error : axe des ordonnées), la MSE étant obtenue selon l'expression (31). Sur la figure 5B, les courbes a, b et c correspondent respectivement aux modèles paramétriques établis respectivement selon les expressions (8), (9), (23). La courbe d correspond à un modèle linéaire (équation (8)), préétabli, avec Z = 1,11977. Il apparaît que les modèles b) et c) semblent particulièrement pertinents.

On va à présent décrire, en lien avec la figure 3, une mise en oeuvre d'un capteur de gaz en utilisant une fonction de calibration telle que précédemment décrite.

Etape 100 : illumination du gaz à un instant de mesure $t$ ;

Etape 110 : mesure de l'intensité de référence $I_{ref}(t)$, dans la bande spectrale de référence $\Delta_{ref}$, par le photo-détecteur de référence $20_{ref}$.

Etape 120 : mesure de l'intensité $I(t)$ du rayonnement 14 transmis par le gaz, dans la bande spectrale de mesure $\Delta_{mes}$, par le photodétecteur de mesure 20.

Etape 130 : estimation d'une intensité $\hat{I}_0(t)$ qui serait détectée par le photodétecteur de mesure 14, dans la bande spectrale de mesure $\Delta_{20}$, en l'absence de gaz dans l'enceinte. L'estimation est effectuée en prenant en compte la fonction de calibration f et en appliquant l'expression :

$$\hat{I}_0(t) = f\big(I_{ref}(t)\big).$$

$$abs(k) = 1 - \frac{I(t)}{\hat{I}_0(t)}$$

Etape 140 : estimation d'une absorption $\qquad$ dans la bande spectrale de mesure $\Delta_{mes}$.

Etape 150 : à partir de l'absorption, estimation d'une quantité $c_x(k)$ d'une espèce gazeuse $G_x$ à partir du ratio en appliquant l'expression (1).

Etape 160 : réitération des étapes 100 à 150, en incrémentant l'instant de mesure $t$, ou sortie de l'algorithme.

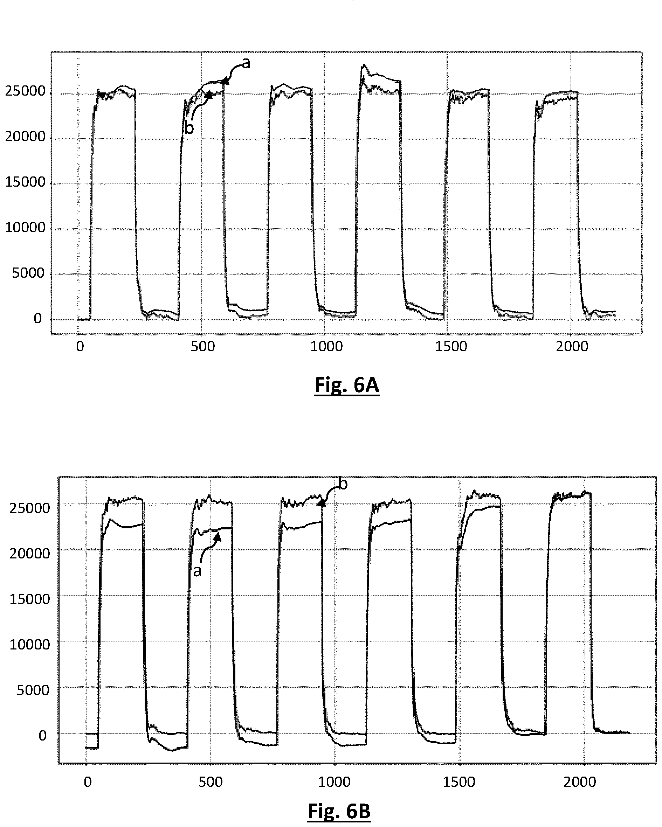

On a mis en oeuvre des capteurs de gaz tel que précédemment cité, en les exposant à une concentration de $CH_4$ alternant entre 25000 ppm et 0 ppm. Les figures 6A et 6B représentent les concentrations mesurées par chaque capteur, en fonction du temps, sans mettre en oeuvre l'invention (courbes a) et en mettant en oeuvre l'invention (courbes b), la fonction de calibration étant de type $f(I_{ref}(k)) = (Z(I_{ref}(k))^{\alpha}$ avec $\alpha = 1,12$. Sur les figures 6A et 6B, l'axe des abscisse correspond au temps (unité - secondes) et l'axe des ordonnées correspond à la concentration mesurée par le capteur. On observe que l'invention (courbes b) permet de se rapprocher davantage des valeurs exactes (25000 ppm ou 0 ppm) par rapport au capteur utilisé sans mise en oeuvre de l'invention (courbes a).

Selon une variante, un capteur de gaz est associé avec un capteur de température 22. Lors de l'établissement de la fonction de calibration f, à chaque mesure de calibration, une température ambiante $T_a$ est mesurée. On établit une fonction de calibration $f_{T_a}$ pour chaque température ambiante. Alternativement, on établit une même fonction de calibration f, en prenant en compte les mesures effectuées pour l'ensemble des températures ambiantes.

Lors des mesures, une mesure de la température ambiante du capteur $T_a(t)$ est mesurée à chaque instant de mesure $t$. On utilise la fonction de calibration $f_{T_a}$ pour laquelle la température ambiante est la plus proche de la température ambiante mesurée.

**Revendications**

1. Procédé de calibration d'un capteur de gaz, le capteur de gaz étant destiné à déterminer une concentration ($c_x$) d'une espèce gazeuse ($G_x$) dans un gaz, l'espèce gazeuse étant apte à absorber une lumière dans une bande spectrale d'absorption ($\Delta_x$), le capteur de gaz comprenant :

- une enceinte (10), configurée pour contenir le gaz ;
- une source de lumière (11), un photodétecteur de mesure (20), et un photodétecteur de référence ($20_{ref}$), la source de lumière (11) étant configurée pour émettre une onde lumineuse incidente (12), l'onde lumineuse incidente se propageant à travers l'enceinte vers le photodétecteur de mesure (20) et vers le photodétecteur de référence ($20_{ref}$) ;
le capteur de gaz étant tel que

- la source de lumière est configurée pour être parcourue par un courant électrique d'alimentation, de façon à porter la source de lumière à une valeur de température ;
- le photodétecteur de mesure est agencé pour mesurer une intensité ($I_{mes}(t)$), dite intensité de mesure, d'une onde lumineuse (14), émise par la source de lumière, transmise par le gaz contenu dans l'enceinte, dans une bande spectrale de mesure ($\Delta_{mes}$), comprenant la bande spectrale d'absorption ($\Delta_x$) ;
- le photodétecteur de référence est agencé pour mesurer une intensité de référence ($I_{ref}(t)$) d'une l'onde lumineuse de référence émise par la source de lumière (11) dans une bande spectrale de référence ($\Delta_{ref}$) ;

le procédé comportant les étapes suivantes :

- (i) prise en compte d'une fonction de calibration (f), non linéaire, la fonction de calibration permettant d'estimer une intensité mesurée ($I_0$), dans la bande spectrale de mesure, par le photodétecteur de mesure, en l'absence d'espèce gazeuse, à partir d'une intensité de référence ($I_{ref}$), mesurée dans la bande spectrale de référence, par le photodétecteur de référence, la fonction de calibration dépendant de paramètres ($\theta$) ;
- (ii) remplissage de l'enceinte par un gaz comportant une concentration nulle, ou considérée comme nulle, de l'espèce gazeuse ;
- (iii) mesure, par le photodétecteur de mesure (20), d'une intensité de mesure ($I_{mes}(k)$), dans la bande spectrale de mesure, et mesure, par le photodétecteur de référence ($20_{ref}$), d'une intensité de référence ($I_{ref}(k)$), dans la bande spectrale de référence, l'étape (iii) étant répétée à différents instants de calibration (k), en modifiant, entre différents instants de calibration, le courant d'alimentation de la source ;
- (iv) à partir des mesures résultant de (iii), détermination des paramètres ($\theta$) de la fonction de calibration.

2. Procédé selon la revendication 1, dans lequel l'étape (iv) comporte :

- iv-1) à partir de chaque intensité de référence ($I_{ref}(k)$) mesurée respectivement à chaque instant de calibration, estimation d'une intensité de mesure ($\hat{I}_0(k)$) résultant du photodétecteur de mesure en utilisant la fonction de calibration ;
- iv-2) comparaison pour chaque valeur du courant d'alimentation, de l'intensité de mesure ($\hat{I}_0(k)$) estimée lors de iv-1) avec l'intensité de mesure ($I_{mes}(k)$) mesurée par le photodétecteur de mesure ;
- iv-3) détermination des paramètres ($\theta$) de la fonction de calibration minimisant les comparaisons résultant de iv-2).

3. Procédé selon l'une quelconque des revendications précédentes, dans lequel lors de l'étape (iii), le courant d'alimentation varie entre deux valeurs extrêmes correspondant respectivement à -15% et +15% d'un courant d'alimentation nominal.

4. Procédé selon l'une quelconque des revendications précédentes, dans lequel

- l'étape (iii) est mise en oeuvre en disposant le capteur de gaz à différentes valeurs d'une température ambiante ($T_a$) ;
- l'étape (iv) est mise en oeuvre en prenant en compte les mesures résultant de (iii) pour chaque valeur de température, de façon à obtenir, une fonction de calibration ($f_{T_a}$) respectivement associée à chaque valeur de température ambiante.

5. Procédé selon l'une quelconque des revendications 1 à 3, dans lequel

- l'étape (iii) est mise en oeuvre en disposant le capteur de gaz à différentes valeurs de température ambiante ;
- l'étape (iv) est mise en oeuvre en prenant en compte les mesures résultant de (iii) pour l'ensemble des valeurs de température, de façon à obtenir, une fonction de calibration commune à l'ensemble des valeurs de température ambiante.

6. Procédé selon l'une quelconque des revendications précédentes, dans lequel un paramètre de la fonction de calibration forme un exposant ($\alpha$) appliqué à l'intensité de référence ($I_{ref}$), le paramètre étant estimé lors de l'étape (iv).

7. Procédé selon la revendication 6, dans lequel un paramètre de la fonction de calibration est un paramètre de proportionnalité (Z), définissant une proportionnalité entre l'intensité estimée ($\hat{I}_0$), dans la bande spectrale de mesure, en l'absence d'espèce gazeuse, et l'intensité de référence ($I_{ref}$) à laquelle l'exposant est appliqué, le paramètre de proportionnalité étant estimé lors de l'étape (iv).

8. Procédé selon l'une quelconque des revendications précédentes, dans lequel la fonction de calibration comporte un ratio d'un numérateur sur un dénominateur, de telle sorte que

- le numérateur comporte un premier paramètre multiplicatif appliqué à l'intensité de référence ;
- le dénominateur comporte un deuxième paramètre multiplicatif appliqué à l'intensité de référence ;
- le premier paramètre multiplicatif et le deuxième paramètre multiplicatif sont estimés lors de l'étape (iv).

9. Procédé de mesure d'une quantité ($c_x$) d'une espèce gazeuse ($G_x$) présente dans un gaz, l'espèce gazeuse étant apte à absorber une lumière dans une bande spectrale d'absorption ($\Delta_x$) le procédé comportant les étapes suivantes :

- a) disposition du gaz entre une source de lumière (11) et un photodétecteur de mesure (20), la source de lumière (11) étant configurée pour émettre une onde lumineuse incidente (12), l'onde lumineuse incidente se propageant à travers le gaz vers le photodétecteur de mesure (20), la source de lumière étant parcourue par un courant électrique d'alimentation, de façon à porter la source de lumière à une valeur de température ;
- b) illumination du gaz *(G)* par la source de lumière (11) ;
- c) mesure, par le photodétecteur de mesure (20), d'une intensité ($I_{mes}(t)$), dite intensité de mesure, d'une onde lumineuse (14) transmise par le gaz, dans une bande spectrale de mesure ($\Delta_{mes}$), comprenant la bande spectrale d'absorption ($\Delta_x$) ;
- d) mesure, par un photodétecteur de référence ($20_{ref}$), d'une intensité ($I_{ref}(t)$), dite intensité de référence, d'une onde lumineuse ($12_{ref}$) de référence, l'onde lumineuse de référence étant émise par la source de lumière (11) dans une bande spectrale de référence ($\Delta_{ref}$) ;

les étapes b) à d) étant mises en oeuvre à un instant de mesure (t), le procédé comportant, à chaque instant de mesure :

- e) à partir de l'intensité de référence ($I_{mes}(t)$) mesurée par le photodétecteur de référence, prise en compte d'une fonction de calibration (f), de façon à estimer une intensité d'une onde lumineuse qui serait détectée par le photodétecteur de mesure, dans la bande spectrale de mesure ($\hat{I}_0(t)$), en l'absence de l'espèce gazeuse ;
- f) estimation d'une quantité ($c_x(t)$) de l'espèce gazeuse ($G_x$), à partir de l'intensité de mesure mesurée lors de l'étape c), de l'intensité estimée lors de l'étape e) ;

le procédé étant **caractérisé en ce que** la fonction de calibration est établie au cours d'une phase de calibration, la phase de calibration étant effectuée en mettant en oeuvre les étapes (i) à (iv) d'un procédé selon l'une quelconque des revendications 1 à 8.

10. Procédé selon la revendication 9, dans lequel la fonction de calibration est établie de telle sorte que :

- l'étape (iii) est mise en oeuvre en disposant le capteur de gaz à différentes valeurs de température ambiante ;
- l'étape (iv) est mise en oeuvre en prenant en compte les mesures résultant de (iii) pour chaque valeur de température, de façon à obtenir, une fonction de calibration respectivement associée à chaque valeur de température ambiante ;
- l'étape e) comporte :

• une prise en compte d'une température ambiante ($T_a$), autour du capteur de gaz ;
• une sélection de la fonction de calibration ($f_{T_a}$) en fonction de la température ambiante.

11. Capteur de gaz, destiné à déterminer une concentration d'une espèce gazeuse dans un gaz, l'espèce gazeuse étant apte à absorber une lumière dans une bande spectrale d'absorption ($\Delta_x$), le capteur comprenant

- une enceinte (10), configurée pour contenir le gaz ;
- une source de lumière (11), un photodétecteur de mesure (20), et un photodétecteur de référence ($20_{ref}$), la source de lumière (11) étant configurée pour émettre une onde lumineuse incidente (12), l'onde lumineuse incidente se propageant à travers l'enceinte vers le photodétecteur de mesure (20) et vers le photodétecteur de référence ($20_{ref}$) ;

le capteur étant tel que

- la source de lumière est parcourue par un courant électrique d'alimentation, de façon à porter la source de lumière à une valeur de température ;
- le photodétecteur de mesure est configuré pour mesurer une intensité, dite intensité de mesure, d'une onde lumineuse (14), émise par la source de lumière (11), transmise par le gaz, contenu dans l'enceinte, dans une bande spectrale de mesure, comprenant la bande spectrale d'absorption ($\Delta_x$) ;
- le photodétecteur de référence étant configuré pour mesurer une intensité de référence ($I_{ref}(t)$) d'une l'onde lumineuse de référence émise par la source de lumière (11) dans une bande spectrale de référence ($\Delta_{ref}$) ;
- le capteur comporte une unité de traitement (30), programmée pour stocker une fonction de calibration non linéaire établie selon l'une quelconque des revendications 1 à 8 et pour appliquer la fonction de calibration à l'intensité de référence mesurée par le photodétecteur de mesure.

12. Capteur de gaz selon la revendication 11, comportant un capteur de température (22), configuré pour mesurer une température ambiante ($T_a(t)$) à l'instant de mesure, l'unité de traitement étant programmée pour sélectionner la fonction de calibration ($f_{T_a}$) en fonction de la température ambiante, parmi plusieurs fonctions de calibration respectivement associées à différentes températures ambiantes.


**Patentansprüche**

1. Verfahren zur Kalibrierung eines Gassensors, wobei der Gassensor dazu bestimmt ist, eine Konzentration ($c_x$) einer gasförmigen Spezies ($G_x$) in einem Gas zu bestimmen, wobei die gasförmige Spezies geeignet ist, Licht in einem Absorptionsspektralband ($\Delta_x$) zu absorbieren, der Gassensor umfassend:

   - eine Kammer (10), die so konfiguriert ist, dass sie das Gas enthält;
   - eine Lichtquelle (11), einen Messphotodetektor (20) und einen Bezugsphotodetektor ($20_{ref}$), wobei die Lichtquelle (11) so konfiguriert ist, dass sie eine einfallende Lichtwelle (12) emittiert, wobei sich die einfallende Lichtwelle durch die Kammer zu dem Messphotodetektor (20) und zu dem Bezugsphotodetektor ($20_{ref}$) hin ausbreitet;
   wobei der Gassensor so beschaffen ist, dass

   - die Lichtquelle so konfiguriert ist, dass sie von einem elektrischen Versorgungsstrom durchflossen wird, um die Lichtquelle auf einen Temperaturwert zu bringen;
   - wobei der Messphotodetektor so eingerichtet ist, dass er eine Intensität ($I_{mes}(t)$), die sogenannte Messintensität, einer Lichtwelle (14), die von der Lichtquelle emittiert und durch das in der Kammer enthaltene Gas übertragen wird, in einem Messspektralband ($\Delta_{mes}$), das das Absorptionsspektralband ($\Delta_x$) umfasst, misst;
   - der Bezugsphotodetektor so eingerichtet ist, dass er eine Bezugsintensität ($I_{ref}(t)$) einer Bezugslichtwelle misst, die von der Lichtquelle (11) in einem Bezugsspektralband ($\Delta_{ref}$) emittiert wird;

   wobei das Verfahren die folgenden Schritte aufweist:

   - (i) Berücksichtigung einer nichtlinearen Kalibrierungsfunktion (f), wobei die Kalibrierungsfunktion es ermöglicht, eine gemessene Intensität ($I_0$) in dem Messspektralband durch den Messphotodetektor in Abwesenheit einer gasförmigen Spezies aus einer Bezugsintensität ($I_{ref}$) zu schätzen, die in dem Bezugsspektralband durch den Bezugsphotodetektor gemessen wurde, wobei die Kalibrierungsfunktion von Parametern ($\theta$) abhängt;
   - (ii) Auffüllung der Kammer mit einem Gas, das eine Konzentration der gasförmigen Spezies von null oder als null betrachtet aufweist,
   - (iii) Messen einer Messintensität ($I_{mes}(k)$) in dem Messspektralband durch den Messphotodetektor (20) und Messen einer Bezugsintensität ($I_{ref}(k)$) in dem Bezugsspektralband durch den Bezugsphotodetektor ($20_{ref}$), wobei Schritt (iii) zu verschiedenen Kalibrierungszeitpunkten (k) wiederholt wird, indem zwischen verschiedenen Kalibrierungszeitpunkten der Versorgungsstrom der Quelle geändert wird;
   - (iv) aus den Messungen, die sich aus (iii) ergeben, Bestimmung der Parameter ($\theta$) der Kalibrierungsfunktion.

2. Verfahren nach Anspruch 1, wobei Schritt (iv) aufweist:

   - iv-1) aus jeder Bezugsintensität ($I_{ref}(k)$), die jeweils zu jedem Kalibrierungszeitpunkt gemessen wurde, Schätzung einer Messintensität ($\hat{I}_0(k)$), die sich aus dem Messphotodetektor ergibt, unter Verwendung der Kalibrierungsfunktion;
   - iv-2) Vergleich für jeden Wert des Versorgungsstroms der in iv-1) geschätzten Messintensität ($\hat{I}_0(k)$) mit der vom Messphotodetektor gemessenen Messintensität ($I_{mes}(k)$);
   - iv-3) Bestimmung der Parameter ($\theta$) der Kalibrierungsfunktion, die die sich aus iv-2) ergebenden Vergleiche minimiert.

3. Verfahren nach einem der vorhergehenden Ansprüche, wobei in Schritt (iii) der Versorgungsstrom zwischen zwei Extremwerten schwankt, die jeweils -15 % und +15 % eines nominalen Versorgungsstroms entsprechen.

**4.** Verfahren nach einem der vorhergehenden Ansprüche, wobei

- Schritt (iii) ausgeführt wird, indem der Gassensor bei verschiedenen Werten einer Umgebungstemperatur ($T_a$) angeordnet wird;
- Schritt (iv) ausgeführt wird, indem die sich aus (iii) ergebenden Messungen für jeden Temperaturwert berücksichtigt werden, um eine Kalibrierungsfunktion ($f_{T_a}$) zu erhalten, die jeweils jedem Wert der Umgebungstemperatur zugeordnet ist.

**5.** Verfahren nach einem der Ansprüche 1 bis 3, wobei

- Schritt (iii) ausgeführt wird, indem der Gassensor bei verschiedenen Umgebungstemperaturwerten angeordnet wird;
- Schritt (iv) durchgeführt wird, indem die sich aus (iii) ergebenden Messungen für alle Temperaturwerte berücksichtigt werden, um eine Kalibrierungsfunktion zu erhalten, die allen Umgebungstemperaturwerten gemeinsam ist.

**6.** Verfahren nach einem der vorhergehenden Ansprüche, wobei ein Parameter der Kalibrierungsfunktion einen Exponenten ($\alpha$) bildet, der auf die Bezugsintensität ($I_{ref}$) angewendet wird, wobei der Parameter in Schritt (iv) geschätzt wird.

**7.** Verfahren nach Anspruch 6, wobei ein Parameter der Kalibrierungsfunktion ein Proportionalitätsparameter (Z) ist, der eine Proportionalität zwischen der geschätzten Intensität ($\hat{I}_0$) in dem Messspektralband in Abwesenheit einer gasförmigen Spezies und der Bezugsintensität ($I_{ref}$), auf die der Exponent angewendet wird, definiert, wobei der Proportionalitätsparameter in Schritt (iv) geschätzt wird.

**8.** Verfahren nach einem der vorhergehenden Ansprüche, wobei die Kalibrierungsfunktion ein Verhältnis eines Zählers zu einem Nenner aufweist, so dass

- der Zähler einen ersten multiplikativen Parameter aufweist, der auf die Bezugsintensität angewendet wird;
- der Zähler einen zweiten multiplikativen Parameter aufweist, der auf die Bezugsintensität angewendet wird;
- der erste multiplikative Parameter und der zweite multiplikative Parameter in Schritt (iv) geschätzt werden.

**9.** Verfahren zur Messung einer Menge ($c_x$) einer gasförmigen Spezies ($G_x$), die in einem Gas vorhanden ist, wobei die gasförmige Spezies geeignet ist, Licht in einem Absorptionsspektralband ($\Delta_x$) zu absorbieren, wobei das Verfahren die folgenden Schritte aufweist:

- a) Anordnung des Gases zwischen einer Lichtquelle (11) und einem Messphotodetektor (20), wobei die Lichtquelle (11) so konfiguriert ist, dass sie eine einfallende Lichtwelle (12) emittiert, wobei sich die einfallende Lichtwelle durch das Gas in Richtung des Messphotodetektors (20) ausbreitet, wobei die Lichtquelle von einem elektrischen Versorgungsstrom durchflossen wird, um die Lichtquelle auf einen Temperaturwert zu bringen;
- b) Beleuchtung des Gases (G) mit der Lichtquelle (11),
- c) Messung durch den Messphotodetektor (20) einer Intensität ($I_{mes}(t)$), der sogenannten Messintensität, einer durch das Gas übertragenen Lichtwelle (14) in einem Messspektralband ($\Delta_{mes}$), das das Absorptionsspektralband ($\Delta_x$) umfasst,
- d) Messung durch einen Bezugsphotodetektor ($20_{ref}$) einer Intensität ($I_{ref}(t)$), der sogenannten Bezugsintensität, einer Bezugslichtwelle ($12_{ref}$), wobei die Bezugslichtwelle von der Lichtquelle (11) in einem Bezugsspektralband ($\Delta_{ref}$) emittiert wird;

wobei die Schritte b) bis d) zu einem Messzeitpunkt (t) ausgeführt werden, das Verfahren zu jedem Messzeitpunkt aufweisend:

- e) ausgehend von der durch den Bezugsphotodetektor gemessenen Bezugsintensität ($I_{mes}(t)$), Berücksichtigung einer Kalibrierungsfunktion (f), um eine Intensität einer Lichtwelle zu schätzen, die durch den Messphotodetektor in dem Messspektralband ($I_0(t)$) in Abwesenheit der gasförmigen Spezies detektiert werden würde;
- f) Schätzung einer Menge ($c_x(t)$) der gasförmigen Spezies ($G_x$) aus der in Schritt c) gemessenen Messintensität, der in Schritt e) geschätzten Intensität;

wobei das Verfahren **dadurch gekennzeichnet ist, dass** die Kalibrierungsfunktion während einer Kalibrierungsphase festgelegt wird, wobei die Kalibrierungsphase durch Ausführen der Schritte (i) bis (iv) eines Verfahrens nach einem der Ansprüche 1 bis 8 durchgeführt wird.

10. Verfahren nach Anspruch 9, bei dem die Kalibrierungsfunktion so festgelegt wird, dass:

- Schritt (iii) ausgeführt wird, indem der Gassensor bei verschiedenen Umgebungstemperaturwerten angeordnet wird;
- Schritt (iv) ausgeführt wird, indem die sich aus (iii) ergebenden Messungen für jeden Temperaturwert berücksichtigt werden, um eine Kalibrierungsfunktion zu erhalten, die jeweils jedem Wert der Umgebungstemperatur zugeordnet ist,
- Schritt e) aufweist:

  • eine Berücksichtigung einer Umgebungstemperatur ($T_a$) um den Gassensor herum;
  • eine Auswahl der Kalibrierungsfunktion ($f_{T_a}$) in Abhängigkeit von der Umgebungstemperatur.

11. Gassensor zum Bestimmen einer Konzentration einer gasförmigen Spezies in einem Gas, wobei die gasförmige Spezies geeignet ist, Licht in einem Absorptionsspektralband ($\Delta_x$) zu absorbieren, der Sensor umfassend

- eine Kammer (10), die so konfiguriert ist, dass sie das Gas enthält;
- eine Lichtquelle (11), einen Messphotodetektor (20) und einen Bezugsphotodetektor ($20_{ref}$), wobei die Lichtquelle (11) so konfiguriert ist, dass sie eine einfallende Lichtwelle (12) emittiert, wobei sich die einfallende Lichtwelle durch die Kammer zu dem Messphotodetektor (20) und zu dem Bezugsphotodetektor ($20_{ref}$) hin ausbreitet;

wobei der Sensor so beschaffen ist, dass

- die Lichtquelle von einem elektrischen Versorgungsstrom durchflossen wird, um die Lichtquelle auf einen Temperaturwert zu bringen;
- der Messphotodetektor so konfiguriert ist, dass er eine Intensität, die sogenannte Messintensität, einer Lichtwelle (14) misst, die von der Lichtquelle (11) emittiert wird, die von dem Gas, das in der Kammer enthalten ist, in einem Messspektralband, das das Absorptionsspektralband ($\Delta_x$) umfasst, übertragen wird;
- der Bezugsphotodetektor so konfiguriert ist, dass er eine Bezugsintensität ($I_{ref}(t)$) einer Bezugslichtwelle misst, die von der Lichtquelle (11) in einem Bezugsspektralband ($\Delta_{ref}$) emittiert wird;
- der Sensor eine Verarbeitungseinheit (30) aufweist, die so programmiert ist, dass sie eine nach einem der Ansprüche 1 bis 8 festgelegte nichtlineare Kalibrierungsfunktion speichert und die Kalibrierungsfunktion auf die von dem Messphotodetektor gemessene Bezugsintensität anwendet.

12. Gassensor nach Anspruch 11, aufweisend einen Temperatursensor (22), der so konfiguriert ist, dass er eine Umgebungstemperatur ($T_a(t)$) zu dem Messzeitpunkt misst, wobei die Verarbeitungseinheit so programmiert ist, dass sie die Kalibrierungsfunktion ($f_{T_a}$) in Abhängigkeit von der Umgebungstemperatur aus mehreren Kalibrierungsfunktionen auswählt, die jeweils verschiedenen Umgebungstemperaturen zugeordnet sind.

**Claims**

1. A method for calibrating a gas sensor, the gas sensor being intended to determine a concentration ($c_x$) of a gaseous species ($G_x$) in a gas, the gaseous species being able to absorb light in an absorption spectral band ($\Delta_x$), the gas sensor comprising:

- a chamber (10) configured to contain the gas;
- a light source (11), a measurement photodetector (20) and a reference photodetector ($20_{ref}$), the light source (11) being configured to emit an incident light beam (12), the incident light beam traveling through the chamber toward the measurement photodetector (20) and toward the reference photodetector ($20_{ref}$);
the gas sensor being such that

  - the light source is configured to have a supply electric current passing through it so as to raise the light source to a temperature value;
  - the measurement photodetector is designed to measure an intensity ($I_{mes}(t)$), referred to as measurement intensity, of a light beam (14) emitted by the light source, transmitted by the gas contained in the chamber, in a measurement spectral band ($\Delta_{mes}$) comprising the absorption spectral band (($\Delta_x$);
- the reference photodetector is designed to measure a reference intensity ($I_{ref}(t)$) of a reference light beam emitted by the light source (11) in a reference spectral band ($\Delta_{ref}$);

the method comprising the following steps:

- (i) incorporating a calibration function ($f$), that is nonlinear, the calibration function making it possible to estimate an intensity ($I_0$), measured in the measurement spectral band by the measurement photodetector in the absence of gaseous species, from a reference intensity ($I_{ref}$) measured in the reference spectral band by the reference photodetector, the calibration function being dependent on parameters ($\theta$);
- (ii) filling the chamber with a gas containing a zero concentration, or concentration considered to be zero, of the gaseous species;
- (iii) having the measurement photodetector (20) measure a measurement intensity ($I_{mes}(k)$) in the measurement spectral band, and having the reference photodetector ($20_{ref}$) measure a reference intensity ($I_{ref}(k)$) in the reference spectral band, step (iii) being repeated at various calibration instants (k) with the source supply current being modified between different calibration instants; (iv) from the measurements resulting from (iii), determining the parameters ($\theta$) of the calibration function.

2. The method as claimed in claim 1, wherein step (iv) includes:

- iv-1) from each reference intensity ($I_{ref}(k)$) measured at each respective calibration instant, estimating a measured intensity ($\hat{I}_0(k)$) resulting from the measurement photodetector using the calibration function;
- iv-2) for each supply current value, comparing the measured intensity ($\hat{I}_0(k)$) estimated during iv-1) with the measured intensity ($I_{mes}(k)$) measured by the measurement photodetector;
- iv-3) determining the parameters ($\theta$) of the calibration function that minimize the comparisons resulting from iv-2).

3. The method as claimed in either one of the preceding claims, wherein during step (iii), the supply current varies between two extreme values corresponding respectively to -15% and +15% of a nominal supply current.

4. The method as claimed in any one of the preceding claims, wherein

- step (iii) is carried out by bringing the gas sensor to different values of an ambient temperature ($T_a$);
- step (iv) is carried out incorporating the measurements resulting from (iii) for each temperature value so as to obtain a calibration function ($f_{Ta}$) associated with each respective ambient temperature value.

5. The method as claimed in any one of claims 1 to 3, wherein

- step (iii) is carried out by bringing the gas sensor to different ambient temperature values;
- step (iv) is carried out incorporating the measurements resulting from (iii) for all of the temperature values so as to obtain a calibration function that is common to all of the ambient temperature values.

6. The method as claimed in any one of the preceding claims, wherein one parameter of the calibration function forms an exponent ($\alpha$) applied to the reference intensity ($I_{ref}$), the parameter being estimated during step (iv).

7. The method as claimed in claim 6, wherein one parameter of the calibration function is a proportionality parameter (Z) defining a proportionality between the estimated intensity ($\hat{I}_0$) in the measurement spectral band in the absence of gaseous species, and the reference intensity ($I_{ref}$) to which the exponent is applied, the proportionality parameter being estimated during step (iv).

8. The method as claimed in any one of the preceding claims, wherein the calibration function comprises a ratio of a numerator over a denominator, such that

- the numerator comprises a first multiplicative parameter applied to the reference intensity;
- the denominator comprises a second multiplicative parameter applied to the reference intensity;
- the first multiplicative parameter and the second multiplicative parameter are estimated during step (iv).

9. A method for measuring a quantity ($c_x$) of a gaseous species ($G_x$) present in a gas, the gaseous species being able to absorb light in an absorption spectral band ($\Delta_x$), the method comprising the following steps:

   - a) placing the gas between a light source (11) and a measurement photodetector (20), the light source (11) being configured to emit an incident light beam (12), the incident light beam traveling through the gas toward the measurement photodetector (20), a supply electric current being passed through the light source so as to raise the light source to a temperature value;
   - b) illuminating the gas (G) using the light source (11);
   - c) having the measurement photodetector (20) measure an intensity ($I_{mes}(t)$), referred to as measured intensity, of a light beam (14) transmitted by the gas, in a measurement spectral band ($\Delta_{mes}$) comprising the absorption spectral band ($\Delta_x$);
   - d) having a reference photodetector ($20_{ref}$) measure an intensity ($I_{ref}(t)$), referred to as reference intensity, of a reference light beam ($12_{ref}$), the reference light beam being emitted by the light source (11) in a reference spectral band ($\Delta_{ref}$);

   steps b) to d) being carried out at a measurement instant (t), the method comprising, at each measurement instant:

   - e) from the reference intensity ($I_{mes}(t)$) measured by the reference photodetector, incorporating a calibration function (f) so as to estimate a light beam intensity that would be detected by the measurement photodetector in the measurement spectral band ($\hat{I}_0(t)$) in the absence of gaseous species;
   - f) estimating a quantity ($c_x(t)$) of the gaseous species ($G_x$) from the measured intensity measured during step c) of the intensity estimated during step e);

   the method being **characterized in that** the calibration function is established during a calibration phase, the calibration phase being performed by implementing steps (i) to (iv) of a method as claimed in any one of claims 1 to 8.

10. The method as claimed in claim 9, wherein the calibration function is established in such a way that:

   - step (iii) is carried out by bringing the gas sensor to different values ambient temperature values;
   - step (iv) is carried out incorporating the measurements resulting from (iii) for each temperature value so as to obtain a calibration function associated with each respective ambient temperature value;
   - step e) comprises:

      • incorporating an ambient temperature ($T_a$), around the gas sensor;
      • selecting the calibration function ($f_{Ta}$) as a function of the ambient temperature.

11. A gas sensor intended to determine a concentration of a gaseous species in a gas, the gaseous species being able to absorb light in an absorption spectral band ($\Delta_x$), the sensor comprising:

   - a chamber (10) configured to contain the gas;
   - a light source (11), a measurement photodetector (20) and a reference photodetector ($20_{ref}$), the light source (11) being configured to emit an incident light beam (12), the incident light beam traveling through the chamber toward the measurement photodetector (20) and toward the reference photodetector ($20_{ref}$);

   the gas sensor being such that

   - an electric supply current passes through the light source so as to raise the light source to a temperature value;
   - the measurement photodetector is designed to measure an intensity, referred to as measurement intensity, of a light beam (14) emitted by the light source (11), transmitted by the gas contained in the chamber, in a measurement spectral band comprising the absorption spectral band ($\Delta_x$);
   - the reference photodetector being designed to measure a reference intensity ($I_{ref}(t)$) of a reference light beam emitted by the light source (11) in a reference spectral band ($\Delta_{ref}$);
   - the sensor comprises a processing unit (30), programmed to store a nonlinear calibration function established as claimed in any one of claims 1 to 8 and to apply the calibration function to the reference intensity measured by the measurement photodetector.

12. The gas sensor as claimed in claim 11, comprising a temperature sensor (22), configured to measure an ambient

temperature ($T_a(t)$) at the measurement instant, the processing unit being programmed to select the calibration function ($f_{Ta}$) as a function of the ambient temperature from among a plurality of calibration functions respectively associated with different ambient temperatures.

**Fig. 1A**

**Fig. 1B**

**Fig. 2A**

**Fig. 2B**

**Fig. 3**

**Fig. 4A**

**Fig. 4B**

**Fig. 5A**

**Fig. 5B**

**Fig. 6A**

**Fig. 6B**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5026992 A
- WO 2007064370 A
- WO 2019145649 A1
- EP 3593119 A